(19) Europäisches Patentamt
European Patent Office
Office européen des brevets

(11) Veröffentlichungsnummer : **0 307 361 B1**

(12) # EUROPÄISCHE PATENTSCHRIFT

(45) Veröffentlichungstag der Patentschrift : **08.04.92 Patentblatt 92/15**

(51) Int. Cl.$^5$ : **C07C 267/00,** C07D 213/72, C07D 295/12

(21) Anmeldenummer : **88810605.1**

(22) Anmeldetag : **05.09.88**

(54) **Verfahren zur Herstellung von Carbodiimiden.**

(30) Priorität : **09.09.87 CH 3483/87**

(43) Veröffentlichungstag der Anmeldung : **15.03.89 Patentblatt 89/11**

(45) Bekanntmachung des Hinweises auf die Patenterteilung : **08.04.92 Patentblatt 92/15**

(84) Benannte Vertragsstaaten : **BE CH DE FR GB IT LI NL**

(56) Entgegenhaltungen :
**DE-A- 823 445**
**CURRENT SCIENCE, Band 54, Nr. 7, 5. April 1985, Seiten 340-342; R. DUBEY et al.: "Dysensitized photo-oxygenation of sym-diphenylthiourea"**

(56) Entgegenhaltungen :
**HOUBEN-WEYL: "Methoden der Organischen Chemie", Band E4, 1983, Seiten 888-896, Georg Thieme Verlag, Stuttgart, DE**
**CHEMICAL ABSTRACTS, Band 76, 1972, Seite 92, Zusammenfassung Nr. 73935n, Columbus, Ohio, US; J.P. DUBOSC et al.: "Photosensitized oxidation of allylthiourea in rigid macromolecular media"**
**CHEMICAL ABSTRACTS, Band 100, 1984, Seite 451, Zusammenfassung Nr. 22218n, Columbus, Ohio, US; H. AOYAMA et al.: "Photochemical reactions of N-thioaroylureas and N-thioroylthioureas"**

(73) Patentinhaber : **CIBA-GEIGY AG**
**Klybeckstrasse 141**
**CH-4002 Basel (CH)**

(72) Erfinder : **Alder, Alex, Dr.**
**Häglerstrasse 22**
**CH-4422 Arisdorf (CH)**

EP 0 307 361 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Carbodiimiden durch Photooxidation von N,N′-substituierten Thioharnstoffen mit Sauerstoff in Gegenwart eines Säurefängers.

In Organic Chemistry, Organic Functional Group Preparations, Vol. 12-II, Seiten 233 bis 259 wird die Herstellung von N,N-substituierten Carbodiimiden durch Desulfurierung von Thioharnstoffen mit z.B. Metalloxiden (HgO, $Ag_2O$, PbO) beschrieben. Die Desulfurierung kann auch mit Alkalihypochloriten in wässrig-alkalischer Lösung vorgenommen werden (siehe auch DE-A-823 445).

Eine Reihe von weiteren Desulfurierungsverfahren für Thioharnstoffe werden in Houben-Weyl, Methoden der organischen Chemie, Bd. E4, 1983, Seiten 890 bis 896, beschrieben. Als Desulfurierungsmittel finden danach Schwermetallverbindungen wie HgO, Oxidationsmittel wie Alkalimetallchlorite oder Chlor, oder andere Reagenzien wie 2-Chlorpyridiniumsalze, Dicarbonylazene oder Triphenylphosphan Verwendung.

R. Dubey et al beschreiben in Current Science, Vol. 54, No. 7, Seiten 340 - 342 (1985) die Photooxidation von N,N′-Diphenylharnstoff mit Singulettsauerstoff in methanolischer Lösung. Hierbei wird 1-Nitrobenzylhydroperoxid gebildet.

Es wurde gefunden, dass man dann Carbodiimide erhält, wenn die Photooxidation von N,N′-substituierten Harnstoffen in Gegenwart eines Säurefängers durchgeführt wird.

Gegenstand der Erfindung ist ein Verfahren zur Herstellung von Carbodiimiden der Formel I

$$R\text{-}N = C = N\text{-}R^1 \quad (I),$$

worin

R und $R^1$ unabhängig voneinander lineares oder verzweigtes $C_1$-$C_{20}$-Alkyl, $C_2$-$C_{18}$-Alkenyl oder $C_2$-$C_{18}$-Alkinyl; $C_3$-$C_{10}$-Cycloalkyl oder -Cycloalkenyl, über ein C-Atom gebundenes Heterocycloalkyl oder Heterocycloalkenyl mit 3 bis 10 Ringatomen, einen polycyclischen Kohlenwasserstoffrest mit 6 bis 10 C-Atomen, $C_6$-$C_{14}$-Aryl, $C_7$-$C_{20}$-Aralkyl, Heteroaryl mit 5 oder 6 Ringatomen, Heteroaralkyl mit 5 oder 6 Ringatomen und 1 bis 6 C-Atomen in der Alkylgruppe, bedeuten, die unsubstituiert oder mit $C_1$-$C_{12}$-Alkyl, -Alkoxy oder Alkylthio, $C_3$-$C_6$-Cycloalkyl, -Cycloalkoxy oder -Cycloalkylthio, $C_6$-$C_{10}$-Aryl, -Aryloxy oder -Arylthio, $C_7$-$C_{16}$-Aralkyl, -Aralkoxy oder -Aralkylthio, Heteroaryl oder Heteroaryloxy mit 5 oder 6 Ringatomen, Cyano, Halogen, $C_2$-$C_{24}$-Sekundäramino, -C(O)$OR^2$, -O(O)$CR^4$, -$NR^2$(O)$CR^4$, -C(O)$NR^2R^3$, worin $R^2$ $C_1$-$C_{12}$-Alkyl, Phenyl oder Benzyl, darstellt, $R^3$ H ist oder die Bedeutung von $R^2$ und $R^4$ die Bedeutung von $R^2$ haben, substituiert sind, wobei die Substituenten Alkyl, Alkoxy und Alkylthio ihrerseits mit $C_1$-$C_{12}$-Alkoxy, Halogen, Cyano, $C_2$-$C_{24}$-Sekundäramino, -C(O)$OR^2$, -O(O)$CR^4$, -$NR^2$(O)$CR^4$, -C(O)$NR^2R^3$, Cycloalkyl oder Heterocycloalkyl mit 4 - 8 Ring-C-Atomen bzw. 5 oder 6 Ringatomen, und die Substituenten Cycloalkyl, Cycloalkoxy, Cycloalkylthio, Aryl, Aryloxy, Arylthio, Aralkyl, Aralkoxy, Aralkylthio, Heteroaryl oder Heteroaryloxy mit $C_1$-$C_{12}$-Alkyl, -Alkoxy oder -Alkylthio, Halogen, Cyano, $C_2$-$C_{24}$-Sekundäramino, -C(O)$OR^2$, -O(O)$CR^4$, -$NR^2$(O)$CR^4$ oder -C(O)$NR^2R^3$ substituiert sein können, und R und $R^1$ in der Bedeutung von Cycloalkyl, Cycloalkenyl, Heterocycloalkyl oder Heterocycloalkenyl mit $C_6$-$C_{14}$-Aryl oder Heteroaryl mit 5 oder 6 Ringatomen kondensiert sein können, durch Photooxidation eines Thioharnstoffs der Formel II

$$R\text{-}HN\text{-}\overset{\overset{S}{\|}}{C}\text{-}NH\text{-}R^1 \qquad (II),$$

worin R und $R^1$ die zuvor angegebene Bedeutung haben, mit Sauerstoff in einem Lösungsmittel, das dadurch gekennzeichnet ist, dass man die Umsetzung in Gegenwart eines im Reaktionsmedium löslichen Säurefängers durchführt.

R und $R^1$ können lineares oder verzweigtes Alkyl mit bevorzugt 1 bis 12, besonders 1 bis 6 C-Atomen sein. Beispiele sind Methyl, Ethyl, n- oder i-Propyl, n-, i- oder t-Butyl, 1-, 2- oder 3-Pentyl, 1-, 2- oder 3-Hexyl, 1-, 2-, 3- oder 4-Heptyl, 1-, 2-, 3- oder 4-Octyl, Nonyl, Decyl, Undecyl, Dodecyl, Tetradecyl, Hexadecyl, Octadecyl und Eicosyl. In einer bevorzugten Ausführungsform sind R und $R^1$ $\alpha$-verzweigtes Alkyl.

Bei R und $R^1$ kann es sich um lineares oder verzweigtes Alkenyl mit vorzugsweise 2 bis 12, besonders 2 bis 6 C-Atomen handeln. Es kann z.B. $C_2$-$C_{17}$-Alkenyl-$C_1$-$C_{16}$-Alkyl mit insgesamt 18 C-Atomen entsprechen. Die Alkenylgruppe kann vorzugsweise 2 - 11 und besonders 2 - 5 C-Atome enthalten und die Alkylgruppe vorzugsweise 1 - 10 und besonders 1 - 4 C-Atome. Beispiele sind: Allyl, But-1-en-3-yl, But-1-en-4-yl, But-2-en-4-yl, Pent-1-en-5-yl, Pent-1-en-4-yl, Pent-1-en-3-yl, Pent-2-en-4-yl, Pent-3-en-5-yl, Hex-1-en-6-yl, Hex-2-en-6-yl, Hex-3-en-6-yl, Hex-3-en-2-yl, Hex-3-en-5-yl, Heptenyl, Octenyl, Nonenyl, Decenyl, Undecenyl, Dodecenyl, Tetradecenyl, Hexadecenyl, Octadecenyl.

R und $R^1$ kann lineares oder verzweigtes Alkinylalkyl mit vorzugsweise 2 bis 12, besonders 2 bis 6 C-Atomen sein. Es kann z.B. $C_2$-$C_{17}$-Alkinyl-$C_1$-$C_{16}$-alkyl mit insgesamt 18 C-Atomen sein. Die Alkinylgruppe kann

vorzugsweise 2 - 11 und besonders 2 - 5 C-Atome und die Alkylgruppe bevorzugt 1 - 10 und besonders 1 - 4 C-Atome enthalten. Beispiele sind Propargyl, But-1-in-3-yl, But-2-in-4-yl, Pent-3-in-1-yl, Pent-1-in-3-yl, Pent-1-in-4-yl, Pent-1-in-5-yl, Pent-2-in-4-yl, Pent-2-in-5-yl, Hex-1-in-3-yl oder -4-yl oder -5-yl oder -6-yl, Hex-2-in-4-yl oder -5-yl oder -6-yl, Hex-3-in-5-yl oder-6-yl, Heptinyl, Octinyl, Noninyl, Decinyl, Undecinyl, Dodecinyl, Tetradecinyl, Hexadecinyl, Octadecinyl.

Bei R und $R^1$ kann es sich um gegebenenfalls mit $C_6$-$C_{14}$-Aryl, vorzugsweise Benzol, oder mit Heteroaryl mit 5 oder 6 Ringatomen kondensiertes Cycloalkyl oder Cycloalkenyl mit vorzugsweise 4 bis 8, besonders 5 oder 6 Ring-C-Atomen handeln. Beispiele sind Cyclopropyl, Cyclobutyl, Cyclopentyl, Cyclohexyl, Cycloheptyl, Cyclooctyl, Cyclodecyl, Cycloprop-1-en-3-yl, Cyclobut-1-en-3-yl, Cyclopent-1-en-3-yl, Cyclopent-1-en-4-yl, Cyclohex-1-en-3-yl, Cyclohex-1-en-4-yl, Cyclopentenyl, Cyclooctenyl, Cyclodecenyl.

R und $R^1$ können gegebenenfalls mit $C_6$-$C_{14}$-Aryl, besonders Benzol, oder mit Heteroaryl mit 5 oder 6 Ringatomen kondensiertes Heterocycloalkyl und Heterocycloalkenyl, oder Heteroaryl sein, die z.B. Heteroatome aus der Gruppe O, S und N, vorzugsweise O enthalten. Bei dem N-Atom handelt es sich um ein tertiäres N-Atom. Sofern das N-Atom im Ring als sekundäre Amingruppe vorliegt, ist dieses N-Atom, z.B. $C_1$-$C_{12}$-, besonders $C_1$-$C_4$-alkyliert, phenyliert oder benzyliert. Es kann auch Schutzgruppen enthalten, z.B. $C_1$-$C_4$-Alkoxymethyl oder $R^5_3$ Si-Gruppen, worin $R^5$ $C_1$-$C_{12}$-Alkyl ist. Diese heterocyclischen Reste enthalten bevorzugt 1 bis 3, besonders 1 oder 2 gleiche oder verschiedene Heteroatome. Das Heterocycloalkyl bzw. -alkenyl enthält bevorzugt 5 oder 6 Ringglieder. Beispiele für Heterocyclen, von denen sich R und $R^1$ ableiten können, sind (Schutzgruppen für sekundäre N-Gruppen sind nicht erwähnt): Pyrrolidin, Tetrahydrofuran, Tetrahydrothiophen, Pyrrolin, Dihydrofuran, Dihydrothiophen, Indan, Dihydrocumaron, Dihydrobenzothiophen, Carbazol, Dibenzofuran, Dibenzothiophen, Pyrazolidin, Imidazolidin, Pyrazolin, Imidazolin, Benzimidazolidin, Oxazolidin, Oxazolin, Thiazolidin, Thiazolin, Isooxazolidin, Isooxazolin, Isothiazolidin, Isothiazolin, Benzoxazolidin, Benzisooxazolidin, Benzthiazolidin, 1,2,3- oder 1,2,4-Triazolidin, 1,2,3- oder 1,2,4-Triazolin, 1,2,3- oder 1,2,4-Oxazolidin oder -Oxazolin, Piperidin, Di- und Tetrahydropyridin, Dihydro- und Tetrahydropyran, Di- und Tetrahydrothiopyran, Piperazin, Dehydropiperazin, Morpholin, Thiomorpholin, 1,3- und 1,4-Dioxan, 1,4-Dithian, Azepan, 1,3-Dioxolan, 1,3-Dithiolan, Pyrrol, Indol, Imidazol, Benzimidazol, Furan, Thiophen, Benzofuran, Benzothiophen, Carbazol, Dibenzofuran, Dibenzothiophen, Oxazol, Isooxazol, Thiazol, Isothiazol, Benzoxazol, Benzothiazol, Pyridin, Pyrimidin, Pyrazin, Pyridazin, Chinolin, Isochinolin, Acridin, Chromen, Chroman, Pyran, Thiapyran, Phenazin, Phenoxazin, Phenolthiazin, Purin. R und $R^1$ in der Bedeutung eines Heterocyclus sind über ein C-Atom an die N-Atome in Formel I gebunden.

R und $R^1$ können ein polycyclischer Kohlenwasserstoffrest mit 6 bis 10 C-Atomen sein. Beispiele für solche Kohlenwasserstoffe von den sich R und $R^1$ ableiten können, sind: Bicyclo-[0,0,3]-hexan, Bicyclo-[1,0,3]-hexan, Bicyclo-[2,2,1]-heptan, Bicyclo-[2,2,1]-hepten, Bicyclo-[2,2,2]-octan und Bicyclo-[2,2,2]-octen.

Bei R und $R^1$ kann es sich um $C_6$-$C_{14}$-Aryl handeln. Beispiele sind Phenyl, Naphthyl, Anthracyl, Indenyl, Indanyl, Fluorenyl, Phenanthryl. Bevorzugt sind Phenyl, Naphthyl und Anthracyl.

Bei R und $R^1$ kann es sich um Aralkyl mit vorzugsweise 7 bis 14 C-Atomen handeln. Das Aryl ist bevorzugt Naphthalin und besonders Benzol. Die Alkylgruppe enthält bevorzugt 1 bis 3 C-Atome. Beispiele für Aralkyl sind Benzyl, 1-Phenyleth-1-yl, 1-Phenyleth-2-yl, 1-Phenylprop-1-yl, -2-yl oder -3-yl, 2-Phenylprop-2-yl- oder -1-yl.

R und $R^1$ können Heteroaralkyl sein. Heteroarylreste und Bevorzugungen sind zuvor erwähnt worden. Die Alkylgruppe des Heteroaralkyls enthält bevorzugt 1 - 3 C-Atome und ist z.B. Methyl, 1,1- oder 1,2-Ethyl oder 1,1-, 2,2-, 1,2- oder 1,3-Propyl.

R und $R^1$ können in beliebigen Stellungen durch gleiche oder verschiedene Reste substituiert sein, z.B. mit 1 bis 5, vorzugsweise 1 bis 3 Substituenten.

Geeignete Substituenten für R und $R^1$ sind:

$C_1$-$C_{12}$-, bevorzugt $C_1$-$C_6$- und besonders $C_1$-$C_4$-Alkyl, -Alkoxy oder -Alkylthio, z.B. Methyl, Ethyl, Propyl, n-, i- und t-Butyl, die Isomeren von Pentyl, Hexyl, Octyl, Nonyl, Decyl, Undecyl und Dodecyl, sowie entsprechende Alkoxy- und Alkylthioreste;

$C_3$-$C_6$-, besonders $C_5$- oder $C_6$-Cycloalkyl, -Cycloalkoxy oder -Cycloalkylthio, z.B. Cyclopentyl, Cyclohexyl, Cyclohexyloxy, Cyclohexylthio; Halogen, bevorzugt F und Cl; CN;

$C_6$-$C_{12}$-Aryl, -Aryloxy oder -Arylthio, in denen Aryl bevorzugt für Naphthyl und besonders Phenyl steht, $C_7$-$C_{16}$-Aralkyl, -Aralkoxy und -Aralkylthio, in denen der Arylrest bevorzugt Naphthyl und besonders Phenyl ist und der Alkylenrest linear oder verzweigt ist und 1 bis 10, vorzugsweisse 1 bis 6 und insbesondere 1 - 3 C-Atome enthält, z.B. Benzyl, Naphthylmethyl, 1- oder 2-Phenyleth-1-yl oder -eth-2-yl, 1-, 2- oder 3-Phenyl-prop-1-yl, -prop-2-yl oder -prop-3-yl, besonders bevorzugt ist Benzyl;

Heteroaryl oder Heteroaryloxy mit 5 oder 6 Ringatomen und bevorzugt Heteroatomen aus der Gruppe O, S und N, wobei das N-Atom wie zuvor definiert tertiär ist. Beispiele sind: Pyridyl, Pyrimidyl, Pyrryl, Furyl, Thienyl und Pyridyloxy.

Sekundäramino mit 2 bis 24, vorzugsweise 2 bis 12 und besonders 2 bis 6 C-Atomen, wobei das Sekundäramino bevorzugt 2 Alkylgruppen enthält, z.B. Dimethyl-, Methylethyl-, Diethyl-, Methyl-n-propyl-, Methyl-n-butyl-, Di-n-Propyl-, Di-n-Butyl-, Di-n-Hexylamino;
$-CONR^2R^3$ oder $-NR^2(O)CR^4$, worin $R^3$ H, $R^2$, $R^3$ und $R^4$ unabhängig voneinander $C_1$-$C_{12}$-, vorzugsweise $C_1$-$C_6$- und insbesondere $C_1$-$C_4$-Alkyl, Phenyl oder Benzyl darstellen, wobei das Alkyl linear oder verzweigt sein kann, z.B. Dimethyl-, Methylethyl-, Diethyl-, Methyl-n-propyl-, Ethyl-n-propyl-, Di-n-propyl-, Methyl-n-butyl-, Ethyl-n-butyl-, n-Propyl-n-butyl- und Di-n-butylcarbamoyl, und worin $R^4$ bevorzugt $C_1$-$C_4$-Alkyl, Phenyl oder Benzyl ist;
$-COOR^2$ oder $-O(O)CR^4$, worin $R^2$ und $R^4$ unabhängig voneinander $C_1$-$C_{12}$-, bevorzugt $C_1$-$C_6$-Alkyl, Phenyl oder Benzyl sind, das linear oder verzweigt sein kann, z.B. Methyl, Ethyl, n- und i-Propyl, n-, i- und t-Butyl, und die Isomeren von Pentyl, Hexyl, Heptyl, Octyl, Nonyl, Decyl, Undecyl und Dodecyl, und worin $R^4$ bevorzugt $C_1$-$C_4$-Alkyl, Phenyl oder Benzyl ist.

Der Substituent Alkyl, Alkoxy und Alkylthio kann seinerseits ein- oder mehrfach besonders ein- bis dreifach, mit Halogen, Cyano, $C_2$-$C_{24}$-Sekundäramino, $-C(O)OR^2$, $-O(O)CR^4$, $-NR^2(O)CR^4$, $-C(O)NR^2R^3$, Cycloalkyl oder Heterocycloalkyl mit 4 - 8 Ring-C-Atomen bzw. 5 oder 6 Ringatomen substituiert sein. Für $R^2$, $R^3$ und $R^4$ gelten die zuvor beschriebenen Bevorzugungen. Ist das Alkyl, Alkoxy oder Alkylthio mit Halogen, vorzugsweise F und-/oder Cl, substituiert, so kann es sich z.B. um $C_1$-$C_6$-, vorzugsweise $C_1$-$C_4$-Haloalkyl handeln, z.B. Trifluor- oder Trichlormethyl, Difluorchlormethyl, Fluordichlormethyl, 1,1-Difluoreth-1-yl, 1,1-Dichloreth-1-yl, 1,1,1-Trichlor- oder -Trifluoreth-2-yl, Pentachlorethyl, Pentafluorethyl, 1,1,1-Trifluor-2,2-dichlorethyl, n-Perfluorpropyl, i-Perfluorpropyl, n-Perfluorbutyl, Fluor- oder Chlormethyl, Difluor- oder Dichlormethyl, 1-Fluor- oder -Chlor-eth-2-yl oder -eth-1-yl, 1-, 2- oder 3-Fluor- oder -Chlor-prop-1-yl oder -prop-2-yl oder -prop-3-yl, 1-Fluor- oder -Chlor-but-1-yl, but-2-yl, -but-3-yl oder -but-4-yl, 2,3-Dichlorprop-1-yl, 1-Chlor-2-fluor-prop-3-yl, 2,3-Dichlorbut-1-yl.

Beispiele für mit Cyano substituiertes Alkyl, Alkoxy oder Alkylthio sind Cyanomethyl, 1- oder 2-Cyanoethyl, 1- oder 2-Cyanopropyl und 2-Cyanoethyloxy. Wenn das Alkyl, Alkoxy oder Alkylthio mit $-C(O)OR^2$, $-O(O)CR^4$, $-NR^2(O)CR^4$ oder $-C(O)NR^2R^3$ substituiert ist, so enthält es bevorzugt 1 bis 3 C-Atome. Beispiele sind Methoxy- oder Ethoxycarbonylmethyl, 1- oder 2-Methoxy- oder -Ethoxycarbonylethyl, 1-, 2- oder 3-Methoxy- oder -Ethoxycarbonylpropyl, Acetyloxymethyl, 1-oder 2-Acetyloxyethyl, Dimethylaminocarbonylmethyl oder -ethyl, N-Methylacetylamino, Methoxycarbonylmethoxy, 1-(Methoxycarbonyl)eth-2-oxy.

Wenn das Alkyl, Alkoxy und Alkylthio mit Cycloalkyl oder Heterocycloalkyl substituiert sind, enthält das Cycloalkyl bevorzugt 5 oder 6 Ring-C-Atome und das Heterocycloalkyl 5 oder 6 Ringatome und bevorzugt Heteroatome aus der Gruppe O, N, S, besonders O, wobei das N-Atom tertiär ist. Beispiele sind Cyclohexylmethyl oder -methoxy oder -methylthio, Cyclopentylethyl, Tetrahydrofurylmethyl, Pyrridylmethyl.

Die Substituenten Cycloalkyl, Cycloalkoxy, Cycloalkylthio, Aryl, Aryloxy, Arylthio, Aralkyl, Aralkoxy, Aralkylthio, Heteroaryl und Heteroaryloxy können ihrerseits wie für Alkyl, Alkoxy und Alkylthio definiert substituiert und zusätzlich ein- oder mehrfach, besonders ein- bis dreifach mit $C_1$-$C_{12}$-, besonders $C_1$-$C_6$-Alkyl oder -Alkylthio substituiert sein. Beispiele sind Methylcyclohexyl, -hexoxy und -hexylthio, Methylphenyl, Dimethylphenyl, Methylchlorphenyl, Cyanophenyl, Chlorphenoxy, Dichlorphenoxy, Trifluormethylphenyl oder -phenoxy, Methoxyphenyl oder -phenoxy, Fluor- oder Difluorphenyl oder -phenoxy, Chlorbenzyl oder -benzyloxy, Methyl-oder Dimethylbenzyl, Carbomethoxyphenyl, Methoxybenzyl, Chlor- oder Dichlorpyrridyl, Methylpyrridyl, Methylpyrridyloxy, Chlor- oder Dichlorpyrridyloxy.

Eine bevorzugte Gruppe von Substituenten für R und $R^1$ ist $C_1$-$C_6$-Alkyl, -Halogenalkyl und -Alkoxy, $C_1$-$C_6$-Alkoxy-$C_1$-$C_3$-alkyl und -alkoxy, $C_1$-$C_6$-Cyanoalkyl, $C_1$-$C_4$-Alkyl $O(O)C$-$C_1$-$C_6$-Alkyl, $C_6$-$C_{12}$-Aryl- und -Aryloxy, $C_7$-$C_{16}$-Alkaryl und -Alkaryloxy, Fluor- und/oder Chloraryl und -aryloxy, Trifluormethylaryl und -aryloxy, $C_1$-$C_6$-Alkoxyaryl und -aryloxy, $C_8$-$C_{16}$-Alkaralkyl und -Alkaralkyloxy, Fluor- und/oder Chlor- und/oder Trifluormethyl-$C_7$-$C_{12}$-Aralkyl und -aralkoxy, $C_7$-$C_{12}$-Aralkyl, Pyrridyl, Pyrridyloxy, Fluor- und/oder Chlorpyrridyl und -pyrridyloxy.

In einer bevorzugten Ausführungsform stehen R und $R^1$ in Formel I für gleiche Reste. In einer anderen bevorzugten Ausführungsform bedeutet in Formel I R unsubstituiertes oder substituiertes $C_6$-$C_{10}$-Aryl und $R^1$ lineares oder verzweigtes $C_1$-$C_{12}$-Alkyl.

Eine andere bevorzugte Ausführungsform ist dadurch gekennzeichnet, dass R und $R^1$ in Formel I unabhängig voneinander unsubstituiertes oder substituiertes $C_1$-$C_{12}$-Alkyl, $C_2$-$C_6$-Alkenyl oder -Alkinyl, $C_4$-$C_8$-Cycloalkyl oder -Cycloalkenyl, Heterocycloalkyl oder Heterocycloalkenyl mit 4 bis 8 Ringatomen, ein polycyclischer Rest mit 6 - 10 C-Atomen, $C_6$-$C_{10}$-Aryl, $C_7$-$C_{16}$-Aralkyl, Heteroaryl mit 5 oder 6 Ringatomen oder Heteroaralkyl mit 5 oder 6 Ringatomen und 1 oder 2 C-Atomen in der Alkylgruppe bedeuten.

In einer besonders bevorzugten Ausführungsform stellen R und $R^1$ in Formel I unabhängig voneinander substituiertes oder unsubstituiertes $C_1$-$C_{18}$-Alkyl, $C_2$-$C_{18}$-Alkenyl, $C_3$-$C_{10}$-Cycloalkyl, $C_6$-$C_{14}$-Aryl oder $C_7$-$C_{16}$-Aralkyl dar.

Thioharnstoffe der Formel II sind bekannt oder können nach allgemein bekannten Verfahren hergestellt werden.

Die Photooxidation kann z.B. mit Licht mit einer Wellenlänge von bevorzugt 200 bis 700 nm vorgenommen werden. Bei der Verwendung von UV-Licht z.B. im UV-B-Bereich kann auf die Mitverwendung von Sensibilisatoren verzichtet werden. Es hat sich als zweckmässig erwiesen, dass man die Photooxidation mit UV-Licht oder sichtbarem Licht in Gegenwart eines Sensibilisators durchführt. Geeignete Lichtquellen sind z.B. Sonnenlicht, Halogenlampen, Glühlampen für Belichten von aussen, Natriumdampflampen oder Quecksilberdampflampen (als UV-Lichtquelle).

Geeignete Sensibilisatoren zur Erzeugung von Singulettsauerstoff sind z.B.: Xanthenfarbstoffe (Bengalrosa), Thiazine (Methylenblau), Porphyrine (Tetraphenylporphyrin), Thionin, Eosin, Erythrosin, Phenosafranin, Chlorophyll, Flavine, Thioxanthone, Phthalocyanine, Thiophene, Naphthalinderivate, Phenothiazine, Pyrazolanthrone, Ketocumarine, Azine (Riboflavin), Anthrachinone, Metallozene, Benzophenone, Anthracenderivate. Eine bevorzugte Gruppe ist Methylenblau, Bengalrosa, Tetraphenylporphyrin und Phtalocyanine.

Die Reaktion kann bei einer Temperatur von z.B. -20°C bis 50°C durchgeführt werden, bevorzugt bei Raumtemperatur (etwa 15°C bis 35°C).

Als Lösungsmittel eignen sich inerte organische Lösungsmittel und Lösungsmittelgemische und deren Mischungen mit Wasser. Geeignete Lösungsmittel sind beispielsweise aliphatische, cycloaliphatische und aromatische Kohlenwasserstoffe (Pentan, Hexan, Cyclohexan, Benzol, Toluol), Chlorkohlenwasserstoffe (Methylenchlorid, Chloroform, Tetrachlorkohlenstoff, Tri- oder Tetrachlorethan, Chlorbenzol), Alkohole (Methanol, Ethanol, Ethylenglykolmonomethylether), Ether (Diethylether, Dibutylether, Ethylenglykoldiethylether, Tetrahydrofuran, Dioxan), Ketone (Methylisobutylketon), Ester (Essigsäureethylester), Nitrile (Acetonitril), N,N-disubstituierte Carbonsäureamide und Lactame (Dimethylacetamid, N-Methylpyrrolidon), Sulfone (Tetramethylensulfon). Ein bevorzugtes Lösungsmittel ist ein Gemisch aus Acetonitril und Wasser.

Bei der Reaktion entsteht Schwefelsäure. Es werden daher zweckmässig mindestens 2 Aequivalente, z.B. 2 bis 2,5 Aequivalente des Säurefängers verwendet.

Bei dem Säurefänger kann es sich z.B. um eine Alkali- oder Erdalkalimetallbase, ein Alkalicarbonat oder -hydrogencarbonat oder eine Pufferlösung mit einem pH $\geqq$ 7 handeln. Beispiele sind LiOH, KOH, NaOH, $Ca(OH)_2$, $NaHCO_3$, $CaHCO_3$, $KHCO_3$. Geeignete Puffermischungen mit pH $\geqq$ 7 sind z.B. wässrige Lösungen von Borax/NaCl oder $KH_2PO_4$, $K_3PO_4$, Borax, $NaHCO_3$, $Na_2HPO_4$ oder KCl und NaOH. Bevorzugt wird NaOH verwendet.

Das Verfahren kann so durchgeführt werden, dass man gasförmigen Sauerstoff, z.B. reinen Sauerstoff, Luft oder Sauerstoff im Gemisch mit Inertgasen in das Reaktionsgemisch leitet. Inertgase sind z.B. Stickstoff, Kohlendioxid und Edelgase, z.B. Helium, Neon und Argon. Bevorzugt ist die Verwendung von Singulettsauerstoff, besonders wenn Sensibilisatoren mitverwendet werden.

Das erfindungsgemässe Verfahren kann im einzelnen so durchgeführt werden, dass man die Verbindung der Formel I, den Säurefänger, das Lösungsmittel und gegebenenfalls den Sensibilisator vorlegt und das Gemisch unter Belichtung im offenen System in Gegenwart von Luft gut rührt oder unter Rühren und Belichten Sauerstoff oder ein Sauerstoff/Inertgas-Gemisch durch die Reaktionsmischung leitet.

Die Aufarbeitung des Reaktionsgemisches erfolgt in üblicher Weise, z.B. durch Extraktion, Waschen und Trocknen des Extraktes und Abdestillieren des Lösungsmittels. Die so erhältlichen Carbodiimide der Formel I können durch Destillation, Kristallisation oder chromatographische Methoden weiter gereinigt werden.

Mit dem erfindungsgemässen Verfahren werden die Carbodiimide bei überraschend kurzen Reaktionszeiten in hohen Ausbeuten erhalten. Ein Vorteil ist, dass die Photooxidation in wässrigen Lösungen vorgenommen werden kann.

Die Carbodiimide der Formel I können in an sich bekannter Weise mit Cyanamid in Cyanoguanidine übergeführt werden, die als latente Härter für z.B. Epoxidharze geeignet sind.

Die nachfolgenden Beispiele erläutern die Erfindung näher.

A) Herstellungsbeispiele

Beispiele 1 - 12:

a) 3,0 g (14,4 mMol) N-Phenyl-N'-t-butylthioharnstoff, 1,28 g (32,0 mMol) NaOH, 30 mg (0,2 Mol-%) Bengalrosa werden in einem Gemisch aus 130 ml Acetonitril und 15 ml Wasser gelöst. Unter kräftigen Rühren wird unter Luftatmosphäre während 1,5 Stunden mit einer 100 W Philips Halogenlampe belichtet. Die Lampe wird in einem doppelwandigen wassergekühlten Glasschacht in die Reaktionslösung getaucht. Danach wird mit 4 x 200 ml Pentan extrahiert, die organische Phase 2x mit Wasser und 1x mit gesättigter NaCl-Lösung gewaschen, über $MgSO_4$ getrocknet, am Rotationsverdampfer eingeengt und unter Hochvakuum im Kugelrohr destilliert.

a') Es wird wie in a) verfahren, anstatt von Bengalrosa wird Methylenblau verwendet, und anstelle der Halogenlampe wird eine Natriumdampflampe verwendet.

b) Es wird wie in a) verfahren, anstatt NaOH aber wird Phosphatpuffer mit pH 7 verwendet. Das Verhältnis $CH_3CN$-$H_2O$ ist 2,3 : 1. Die Lösung ist 0,01 molar an Thioharnstoff. Ferner werden 2 Mol-% Bengalrosa verwendet.

c) Es wird wie in b) verfahren, wobei zusätzlich zur wässrigen Acetonitrillösung ($CH_3CN$-$H_2O$ 4:1) Cyclohexan oder Pentan (ca. 40 % des Reaktionsvolumens) zugegeben wird. Die Lösung ist 0,02-0,05 molar an Thioharnstoff, und es werden 1,5 Mol % Bengalrosa verwendet.

Die Ergebnisse sind in Tabelle 1 zusammengefasst.

| Beispiel | $R-N=C=N-R^1$ | | Ausbeute | IR($CHCl_3$) [$cm^{-1}$] |
|---|---|---|---|---|
| | R | $R^1$ | | |
| $1^a$ | 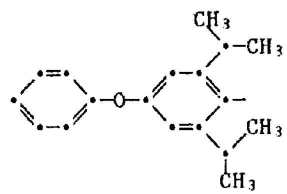 | $t-C_4H_9$ | 91 | 2145 |
| $2^{a'}$ | 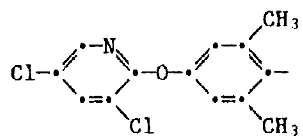 | $t-C_4H_9$ | 88 | 2145 |
| $3^a$ | 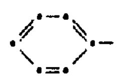 | $t-C_4H_9$ | 83 | 2125 |
| $4^a$ | " | 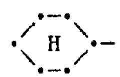 | 71 | 2140 |
| $5^c$ | " | $n-C_4H_9$ | 71 | 2145 |
| $6^b$ | " | $-CH_2CH_2COOCH_3$ | 31 | 1735<br>2145 |

(Fortsetzung)

| Beispiel | $R-N=C=N-R^1$ | | Ausbeute | $IR(CHCl_3)$ $[cm^{-1}]$ |
|---|---|---|---|---|
| | R | $R^1$ | | |
| 7[b] | " | 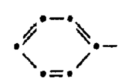 | 37 | 2140 |
| 8[a] | 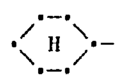 | 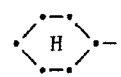 | 57 | 2120 |
| 9[c] | $n-C_4H_9$ | $n-C_4H_9$ | 81 | 2130 |
| 10[b] | 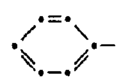 | 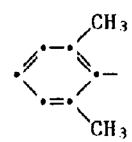 | 66 | 2145 |
| 11[b] | " | $CH_2=CHCH_2-$ | 40 | 2140 |
| 12[b] | $CH_2=CHCH_2-$ | $CH_2=CHCH_2-$ | 41 | 2145 |

(Fortsetzung)

| Beispiel | R-N=C=N-R¹ | | Ausbeute [ % ] | IR($CHCl_3$) [$cm^{-1}$] |
|---|---|---|---|---|
| | R | $R^1$ | | |
| 13[a] | 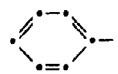 | 1-Adamantyl | 91 | 2110<br>2135 S |
| 14[c] | 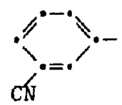 | 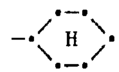 | 46 | 2140 |
| 15[a] | 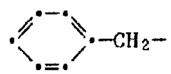 | 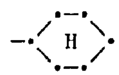 | 58 | 2130 |
| 16[a] | 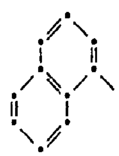 | 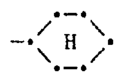 | 22 | 2130 |
| 17[a] | 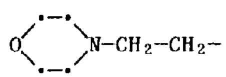 | 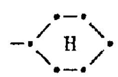 | 53 | 2130 |
| 18[a] | 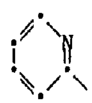 | 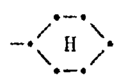 | 41 | 2140 |

(Fortsetzung)

| Beispiel | $R-N=C=N-R^1$ | | Ausbeute [ % ] | $IR(CHCl_3)$ [$cm^{-1}$] |
|---|---|---|---|---|
| | R | $R^1$ | | |
| 19[a] | 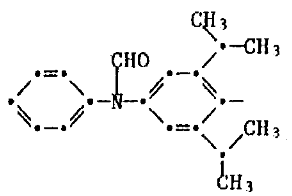 | $t-C_4H_9$ | 74 | 2140<br>1675 |
| 20[a] | 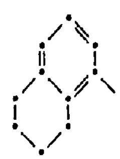 | $t-C_4H_9$ | 87 | 2120 |

Anwendungsbeispiele

Beispiele 21 - 22:

Die Carbodiimide der Beispiele 7 bzw. 8 werden in 1,2-Dichlorethan mit Cyanamid 16 Stunden am Rückfluss erhitzt. Man dampft bis zur Suspension ein, verrührt mit Ether und nutscht die kristallinen Verbindungen ab. Man erhält N-Cyano-N′,N″-diphenylguanidin (I, Schmelzpunkt: 198 - 199°C) bzw. N-Cyano-N′,N″-dicyclohexylguanidin (II, Schmelzpunkt: 191°C).

15 g der Cyanoguanidine werden mit jeweils 100 g eines Bisphenol-A-diglycidylethers (Epoxidgehalt 5,4 Aequivalente/kg) vermischt und die Mischung wird 4 h bei 180°C gehärtet. Dabei entstehen klare Giesskörper mit der in der Tablle 2 angegebenen Glasumwandlungstemperatur (Tg, ermittelt mittels DSC):

| Beispiel | Verbindung Nr. | Tg (°C) |
|---|---|---|
| 21 | I | 145 |
| 22 | II | 129 |

## Patentansprüche

1. Verfahren zur Herstellung von Carbodiimiden der Formel I

$$R\text{-}N = C = N\text{-}R^1 \quad (I),$$

worin
R und $R^1$ unabhängig voneinander lineares oder verzweigtes $C_1$-$C_{20}$-Alkyl, $C_2$-$C_{18}$-Alkenyl oder $C_2$-$C_{18}$-Alkinyl; $C_3$-$C_{10}$-Cycloalkyl oder -Cycloalkenyl, über ein C-Atom gebundenes Heterocycloalkyl oder Heterocycloalkenyl mit 3 bis 10 Ringatomen, einen polycyclischen Kohlenwasserstoffrest mit 6 bis 10 C-Atomen, $C_6$-$C_{14}$-Aryl, $C_7$-$C_{20}$-Aralkyl, Heteroaryl mit 5 oder 6 Ringatomen, Heteroaralkyl mit 5 oder 6 Ringatomen und 1 bis 6 C-Atomen in der Alkylgruppe, bedeuten, die unsubstituiert oder mit $C_1$-$C_{12}$-Alkyl, -Alkoxy oder -Alkylthio, $C_3$-$C_6$-Cycloalkyl, -Cycloalkoxy oder -Cycloalkylthio, $C_6$-$C_{10}$-Aryl, -Aryloxy oder -Arylthio, $C_7$-$C_{16}$-Aralkyl, -Aralkoxy oder -Aralkylthio, Heteroaryl oder Heteroaryloxy mit 5 oder 6 Ringatomen, Cyano, Halogen, $C_2$-$C_{24}$-Sekundäramino, -C(O)OR$^2$, -O(O)CR$^4$, -NR$^2$(O)CR$^4$, -C(O)NR$^2$R$^3$, worin $R^2$ $C_1$-$C_{12}$-Alkyl, Phenyl oder Benzyl, darstellt, $R^3$ H ist oder die Bedeutung von $R^2$ und $R^4$ die Bedeutung von $R^2$ haben, substituiert sind, wobei die Substituenten Alkyl, Alkoxy und Alkylthio ihrerseits mit $C_1$-$C_{12}$-Alkoxy, Halogen, Cyano, $C_2$-$C_{24}$-Sekundäramino, -C(O)OR$^2$, -O(O)CR$^4$, -NR$^2$(O)CR$^4$, -C(O)NR$^2$R$^3$, Cycloalkyl oder Heterocycloalkyl mit 4 - 8 Ring-C-Atomen bzw. 5 oder 6 Ringatomen, und die Substituenten Cycloalkyl, Cycloalkoxy, Cycloalkylthio, Aryl, Aryloxy, Arylthio, Aralkyl, Aralkoxy, Aralkylthio, Heteroaryl oder Heteroaryloxy mit $C_1$-$C_{12}$-Alkyl, -Alkoxy oder -Alkylthio, Halogen, Cyano, $C_2$-$C_{24}$-Sekundäramino, -C(O)OR$^2$, -O(O)CR$^4$, -NR$^2$(O)CR$^4$ oder -C(O)NR$^2$R$^3$ substituiert sein können, und R und $R^1$ in der Bedeutung von Cycloalkyl, Cycloalkenyl, Heterocycloalkyl oder Heterocycloalkenyl mit $C_6$-$C_{14}$-Aryl oder Heteroaryl mit 5 oder 6 Ringatomen kondensiert sein können, durch Photooxidation eines Thioharnstoffs der Formel II

$$R\text{—}HN\text{—}\overset{S}{\overset{\|}{C}}\text{—}NH\text{—}R^1 \quad (II),$$

worin R und $R^1$ die zuvor angegebene Bedeutung haben, mit Sauerstoff in einem Lösungsmittel, dadurch gekennzeichnet, dass man die Umsetzung in Gegenwart eines im Reaktionsmedium löslichen Säurefängers durchführt.

2. Verfahren gemäss Anspruch 1, dadurch gekennzeichnet, dass man die Photooxidation mit UV-Licht oder sichtbarem Licht in Gegenwart eines Sensibilisators durchführt.

3. Verfahren gemäss Anspruch 2, dadurch gekennzeichnet, dass es sich beim Sensibilisator um Methylenblau, Bengalrosa oder Phtalocyanine handelt.

4. Verfahren gemäss Anspruch 1, dadurch gekennzeichnet, dass es bei einer Temperatur von -20°C bis 50°C durchgeführt wird.

5. Verfahren gemäss Anspruch 1, dadurch gekennzeichnet, dass es in organischen Lösungsmitteln, Lösungsmittelgemischen oder deren Mischungen mit Wasser durchgeführt wird.

6. Verfahren gemäss Anspruch 1, dadurch gekennzeichnet, dass man pro Mol Thioharnstoff der Formel II mindestens 2 Aequivalente des Säurefängers verwendet.

7. Verfahren gemäss Anspruch 1, dadurch gekennzeichnet, dass es sich bei dem Säurefänger um eine Alkali- oder Erdalkalimetallbase, ein Alkalicarbonat oder -hydrogencarbonat oder eine Pufferlösung mit einem $pH \geqq 7$ handelt.

8. Verfahren gemäss Anspruch 7, dadurch gekennzeichnet, dass das Lösungsmittel ein Gemisch aus Acetonitril und Wasser ist.

9. Verfahren gemäss Anspruch 1, dadurch gekennzeichnet, dass es mit reinem Sauerstoff, Luft oder einer Mischung von Sauerstoff mit einem Inertgas durchgeführt wird.

10. Verfahren gemäss Anspruch 9, dadurch gekennzeichnet, dass das Inertgas Stickstoff, Kohlendioxid oder ein Edelgas ist.

11. Verfahren gemäss Anspruch 1, dadurch gekennzeichnet, dass R und $R^1$ gleiche Reste sind.

12. Verfahren gemäss Anspruch 1, dadurch gekennzeichnet, dass R und $R^1$ in Formel I unabhängig voneinander unsubstituiertes oder wie in Anspruch 1 definiert substituiertes $C_1$-$C_{12}$-Alkyl, $C_2$-$C_6$-Alkenyl oder -Alkinyl, $C_4$-$C_8$-Cycloalkyl oder -Cycloalkenyl, Heterocycloalkyl oder Heterocycloalkenyl mit 4 bis 8 Ringatomen, ein polycyclischer Rest mit 6 - 10 C-Atomen, $C_6$-$C_{10}$-Aryl, $C_7$-$C_{16}$-Aralkyl, Heteroaryl mit 5 oder 6 Ringatomen oder Heteroaralkyl mit 5 oder 6 Ringatomen und 1 oder 2 C-Atomen die der Alkylgruppe bedeuten.

13. Verfahren gemäss Anspruch 12, dadurch gekennzeichnet, dass in Formel I R unsubstituiertes oder substituiertes $C_6$-$C_{10}$-Aryl und $R^1$ lineares oder verzweigtes $C_1$-$C_{12}$-Alkyl ist.

## Claims

1. A process for the prep,aration of a carbodiimide of the formula I

$$R\text{-}N = C = N\text{-}R^1 \quad (I),$$

in which R and $R^1$ independently of one another are linear or branched $C_1$-$C_{20}$alkyl, $C_2$-$C_{18}$alkenyl or $C_2$-$C_{18}$alkynyl; $C_3$-$C_{10}$cycloalkyl or -cycloalkenyl, heterocycloalkyl or heterocycloalkenyl which has 3 to 10 ring atoms and is bonded via a C atom, a polycyclic hydrocarbon radical with 6 to 10 atoms, $C_6$-$C_{14}$aryl, $C_7$-$C_{20}$aralkyl, heteroaryl with 5 or 6 ring atoms or heteroaralkyl with 5 or 6 ring atoms and 1 to 6 C atoms in the alkyl group, which are unsubstituted or substituted by $C_1$-$C_{12}$alkyl, -alkoxy or alkylthio, $C_3$-$C_6$cycloalkyl, -cycloalkoxy or -cycloalkylthio, $C_6$-$C_{10}$aryl, -aryloxy or -arylthio, $C_7$-$C_{16}$aralkyl, -aralkoxy or -aralkylthio, heteroaryl or heteroaryloxy with 5 or 6 ring atoms, cyano, halogen, $C_2$-$C_{24}$secondary amino, -C(O)OR$^2$, -O(O)CR$^4$, -C(O)NR$^2$R$^3$, in which $R^2$ is $C_1$-$C_{12}$alkyl, phenyl or benzyl, $R^3$ is H or is as defined for $R^2$ and $R^4$ is as defined for $R^2$, it being possible for the substituents alkyl, alkoxy and alkythio in turn to be substituted by $C_1$-$C_{12}$alkoxy, halogen, cyano, $C_2$-$C_{24}$secondary amino, -C(O)OR$^2$, -O(O)CR$^4$, -NR$^2$(O)CR$^4$, -C(O)NR$^2$R$^3$, cycloalkyl or heterocycloalkyl with 4 - 8 ring C atoms or 5 or 6 ring atoms, and for the substituents cycloalkyl, cycloalkoxy, cycloalkylthio, aryl, aryloxy, arylthio, aralkyl, aralkoxy, aralkylthio, heteroaryl or heteroaryloxy to be substituted by $C_1$-$C_{12}$alkyl, -alkoxy or -alkylthio, halogen, cyano, $C_2$-$C_{24}$secondary amino, -C(O)OR$^2$, -O(O)CR$^4$, -NR$^2$(O)CR$^4$ or -C(O)NR$^2$R$^3$, and for cycloalkyl, cyclo-alkyl, cycloalkenyl, heterocycloalkyl or heterocycloalkenyl R and $R^1$ to be fused with $C_6$-$C_{14}$aryl or heteroaryl with 5 or 6 ring atoms, by photooxidation of thiourea of the formula II

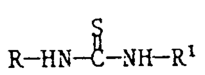

(II),

in which R and $R^1$ are as defined above, with oxygen in a solvent, which comprises carrying out the reaction in the presence of an acid-trapping agent which is soluble in the reaction medium.

2. The process according to claim 1, wherein the photooxidation is carried out with UV light or visible light in the presence of a sensitizer.

3. The process according to claim 2, wherein the sensitizer is methylene blue, Bengal pink or a phthalocyanine.

4. The process according to claim 1, carried out at a temperature of -20°C to 50°C.

5. The process according to claim 1, carried out in an organic solvent, a solvent mixture or a mixture thereof

with water.

6. The process according to claim 1, wherein at least 2 equivalents of the acid-trapping agent are used per mol of thiourea of the formula II.

7. The process according to claim 1, wherein the acid-trapping agent is an alkali metal base or alkaline earth metal base, an alkali metal carbonate or bicarbonate or a buffer solution with a pH $\geqq$ 7.

8. The process according to claim 7, wherein the solvent is a mixture of acetonitrile and water.

9. The process according to claim 1, carried out with pure oxygen, air or a mixture of oxygen with an inert gas.

10. The process according to claim 9, wherein the inert gas is nitrogen, carbon dioxide or a noble gas.

11. The process according to claim 1, wherein R and $R^1$ are identical radicals.

12. The process according to claim 1, wherein R and $R^1$ in formula I independently of one another are unsubstituted or substituted, as defined in claim 1, $C_1$-$C_{12}$alkyl, $C_2$-$C_6$alkenyl or -alkynyl, $C_4$-$C_8$cycloalkyl or -cycloalkenyl, heterocycloalkyl or heterocycloalkenyl with 4 to 8 ring atoms, a polycyclic radical with 6 - 10 C atoms, $C_6$-$C_{10}$aryl, $C_7$-$C_{16}$-aralkyl, heteroaryl with 5 or 6 ring atoms or heteroaralkyl with 5 or 6 ring atoms and 1 or 2 C atoms in the alkyl group.

13. The process according to claim 12, wherein in formula I R is unsubstituted or substituted $C_6$-$C_{10}$aryl and $R^1$ is linear or branched $C_1$-$C_{12}$alkyl.

## Revendications

1. Procédé de préparation des carbodiimides de formule I

$$R\text{-}N = C = N\text{-}R^1 \quad (I),$$

où R et $R^1$ représentent, indépendamment l'un de l'autre, un alkyle en $C_1$-$C_{20}$, un alcényle en $C_2$-$C_{18}$ ou un alcynyle en $C_2$-$C_{18}$ linéaires ou ramifiés, un cycloalkyle en $C_3$-$C_{10}$ ou un cycloalcényle en $C_3$-$C_{10}$, un hétérocycloalkyle ou un hétérocycloalcényle ayant 3 à 10 atomes nucléaires lié par un atome de C, un reste hydrocarboné polycyclique ayant 6 à 10 atomes de C, un aryle en $C_6$-$C_{14}$, un aralkyle en $C_7$-$C_{20}$, un hétéroaryle ayant 5 ou 6 atomes nucléaires, un hétéroaralkyle ayant 5 ou 6 atomes nucléaires et 1 à 6 atomes de C dans le groupe alkyle, qui peuvent être non substitués ou substitués avec un alkyle en $C_1$-$C_{12}$, un alcoxy en $C_1$-$C_{12}$ ou un alkyle en $C_1$-$C_{12}$thio, un cycloalkyle en $C_3$-$C_6$, un cycloalcoxy en $C_3$-$C_6$ ou un cycloalkyle en $C_3$-$C_6$ thio, un aryle en $C_6$-$C_{10}$, un aryle en $C_6$-$C_{10}$ oxy ou un aryle en $C_6$-$C_{10}$ thio, un aralkyle en $C_7$-$C_{16}$, un aralcoxy en $C_7$-$C_{16}$ ou un aralkyle en $C_7$-$C_{16}$ thio, un hétéroaryle ou un hétéroaryloxy ayant 5 ou 6 atomes nucléaires, un cyano, un halogène, un amino secondaire en $C_2$-$C_{24}$, -C(O)O$R^2$, -O(O)C$R^4$, -N$R^2$(O)C$R^4$, -C(O)N$R^2R^3$, $R^2$ représentant un alkyle en $C_1$-$C_{12}$, le phényle ou le benzyle, $R^3$ est H ou a la signification de $R^2$ et $R^4$ a la signification de $R^2$, les substituants alkyle, alcoxy et alkylthio pouvant être, de leur côté, substitués par un alcoxy en $C_1$-$C_{12}$, un halogène, un cyano, un amino secondaire en $C_2$-$C_{24}$, -C(O)O$R^2$, -O(O)C$R^4$, -N$R^2$(O)C$R^4$, -C(O)N$R^2R^3$, un cycloalkyle ou un hétérocycloalkyle ayant 4 à 8 atomes de C nucléaires ou 5 ou 6 atomes nucléaires et les substituants cycloalkyle, cycloalcoxy, cycloalkylthio, aryle, aryloxy, arylthio, aralkyle, aralcoxy, aralkylthio, hétéroaryle ou hétéroaryloxy pouvant être substitués par un alkyle en $C_1$-$C_{12}$, un alcoxy en $C_1$-$C_{12}$ ou un alkyle en $C_1$-$C_{12}$ thio, un halogène, un cyano, un amino secondaire en $C_2$-$C_{24}$, -C(O)O$R^2$, -O(O)C$R^4$, -N$R^2$(O)C$R^4$ ou -C(O)N$R^2R^3$ et R et $R^1$ pouvant être condensés avec un aryle en $C_6$-$C_{14}$ ou un hétéroaryle ayant 5 ou 6 atomes nucléaires lorsqu'ils représentent un cycloalkyle, un cycloalcényle, un hétérocycloalkyle ou un hétérocycloalcényle, par photo-oxydation d'une thiourée de formule II

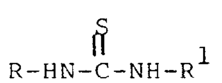

(II),

où R et $R^1$ ont la signification précédemment indiquée, par l'oxygène dans un solvant, caractérisé en ce que l'on effectue la réaction en présence d'un fixateur d'acide soluble dans le milieu réactionnel.

2. Procédé selon la revendication 1, caractérisé en ce que l'on effectue la photo-oxydation avec de la lumière UV ou de la lumière visible en présence d'un sensibilisateur.

3. Procédé selon la revendication 2, caractérisé en ce que le sensibilisateur est du bleu de méthylène, du rose bengale ou des phtalocyanines.

4. Procédé selon la revendication 1, caractérisé en ce qu'il est effectué à une température allant de -20°C à 50°C.

5. Procédé selon la revendication 1, caractérisé en ce qu'il est effectué dans des solvants, mélanges de solvants organiques ou leurs mélanges avec de l'eau.

6. Procédé selon la revendication 1, caractérisé en ce que l'on utilise par mole de thiourée de formule II au moins 2 équivalents du fixateur d'acide.

7. Procédé selon la revendication 1, caractérisé en ce que le fixateur d'acide est une base d'un métal alcalin ou alcalino-terreux, un carbonate ou hydrogénocarbonate alcalin ou une solution tampon ayant un pH $\geqq$ 7.

8. Procédé selon la revendication 7, caractérisé en ce que le solvant est un mélange d'acétonitrile et d'eau.

9. Procédé selon la revendication 1, caractérisé en ce qu'il est effectué avec de l'oxygène pur, de l'air ou un mélange d'oxygène avec un gaz inerte.

10. Procédé selon la revendication 9, caractérisé en ce que le gaz inerte est l'azote, l'anhydride carbonique ou un gaz noble.

11. Procédé selon la revendication 1, caractérisé en ce que R et $R^1$ sont des restes identiques.

12. Procédé selon la revendication 1, caractérisé en ce que R et $R^1$ dans la formule I représentent, indépendamment l'un de l'autre, un alkyle en $C_1$-$C_{12}$, un alcényle en $C_2$-$C_6$ ou un alcynyle en $C_2$-$C_6$, un cycloalkyle en $C_4$-$C_8$ ou un cycloalcényle en $C_4$-$C_8$, un hétérocycloalkyle ou un hétérocycloalcényle ayant 4 à 8 atomes nucléaires, un reste polycyclique ayant 6-10 atomes de C, un aryle en $C_6$-$C_{10}$, un aralkyle en $C_7$-$C_{16}$, un hétéroaryle ayant 5 ou 6 atomes nucléaires ou un hétéroaralkyle ayant 5 ou 6 atomes nucléaires et 1 ou 2 atomes de C dans le groupe alkyle, non substitués ou substitués comme défini dans la revendication 1.

13. Procédé selon la revendication 12, caractérisé en ce que, dans la formule I, R est un aryle en $C_6$-$C_{10}$ non substitué ou substitué et $R^1$ est un alkyle en $C_1$-$C_{12}$ linéaire ou ramifié.